# EUROPEAN PATENT APPLICATION

(11) **EP 4 437 950 A1**
(43) Date of publication of application: **02.10.2024**
(21) Application number: 23165092.0
(22) Date of filing: 29.03.2023
(51) Int. Cl.: A61B 5/08, A61B 5/11, A61B 5/113, A61B 5/00

(54) **DEVICE, SYSTEM AND METHOD FOR SUPPORTING IMPROVED RESPIRATION MONITORING**

(71) Applicant: Koninklijke Philips N.V., 5656 AG Eindhoven (NL)
(72) Inventor: BALMAEKERS, Benoit Marie, 5656 AG Eindhoven (NL); VAN GASTEL, Mark Josephus Henricus, 5656 AG Eindhoven (NL)
(74) Representative: Philips Intellectual Property & Standards

(57) **Abstract**

A device for supporting improved respiration monitoring comprises: an input unit configured to obtain a set of image frames of a region of interest of a patient; a processing unit configured analyze the set of image frames to estimate an amount of motion of the patient in a first direction and in a second direction, wherein the second direction is substantially perpendicular to the first direction, an evaluation unit configured to compare the amount of motion in the first direction with the amount of motion in the second direction and to determine a quality of respiration monitoring based on said comparison; and an output unit configured to provide an quality signal indicating the quality of the respiration monitoring.

## Description

### FIELD OF THE INVENTION

The present invention relates to a device for supporting improved respiration monitoring. The present invention further relates to a corresponding system and method.

### BACKGROUND OF THE INVENTION

Vital signs such as heart rate (HR), respiratory rate (RR), or blood oxygen saturation are important indicators of a person's health and well-being as well as predictors of acute medical conditions and chronic disease states. For this reason, vital signs are widely monitored in hospitalized and outpatient care settings, at home, or in other medical, leisure and fitness settings.

In a clinical environment, observing respiratory activity (breathing frequency) is highly relevant. RR is one of the most important basic vital signs to assess the health status of a patient. In intensive care unit (ICU) settings, respiration is routinely measured via ECG electrodes from an electrocardiogram, and the measured thorax-impedance changes during breathing activity, respectively.

Camera-based measurements of vital signs, particularly RR, allow for contactless measurements, thereby improving patient comfort and reducing the risk of infection since cleaning of contact-probes or the use of single-use electrodes is no longer needed. Furthermore, contactless vital sign measurement can reduce cost of staff time and consumables and reduce environmental impact.

In general, contactless measurements also allow for vital sign measurements in use-cases where vital sign data could provide additional safety or early detection, but is currently not performed due to cost/benefit factors of contact-based methods.

Concerning RR, a (video) camera may capture the breathing movements of a patient's chest or belly in a stream of images. The breathing movements lead to a temporal modulation of certain image features, wherein the frequency of the modulation corresponds to the respiratory rate of the patient monitored. Examples of such image features are the average amplitude in a spatial region of interest located around the patient's chest, or the location of the maximum of the spatial cross correlation of the region of interest in subsequent images.

However, camera-based respiration monitoring is based on detecting subtle respiration motion in a selected region of interest (ROI) in the chest/belly area of the patient. Hence, the quality and the reliability of the obtained vital sign information are largely influenced by the quality of the input image data influenced by an appropriate selection of the image contrast and the selected region of interest. Furthermore, the contactless nature of these measurements comes with an increased sensitivity to subject motion. Particularly respiration rate, despite its importance and the large number of available monitoring techniques, is notoriously difficult to measure when a patient is moving. During periods of motion remote monitoring techniques, based on radar signals, for example, are usually overwhelmed by artifacts and thus ineffective.

For contactless vital sign monitoring, particularly respiration monitoring, metrics may be used to determine when disturbances, such as motion, may prevent accurate measurement. These can then be used to suppress measurements to prevent giving erroneous output. In fact, there exist different methods for filtering disturbances out of a measured signal. However, rather than suppressing erroneous output, it is preferrable to increase the likelihood of correct measurements. A patient may thus be given instructions to remain 'still', but this is ambiguous, and may easily be forgotten, particularly if patients are distressed (as e.g. in an Emergency Department setting).

### SUMMARY OF THE INVENTION

It is an object of the present invention to provide a device for supporting improved respiration monitoring, particularly for supporting higher quality respiration monitoring of patients. Accordingly, it is an object of the present invention to support accurate respiration monitoring.

In a first aspect of the present invention a device for supporting improved respiration monitoring is presented, the device comprising:
an input unit configured to obtain a set of image frames of a region of interest of a patient;
a processing unit configured to analyze the set of image frames to estimate an amount of motion of the patient in a first direction and in a second direction, wherein the second direction is substantially perpendicular to the first direction,
an evaluation unit configured to compare the amount of motion in the first direction with the amount of motion in the second direction and to determine a quality of respiration monitoring based on said comparison; and
an output unit configured to provide a quality signal indicating the quality of the respiration monitoring.

In a second aspect of the present invention a system for supporting improved respiration monitoring is presented, the system comprising:
an imaging device configured to capture a set of image frames of a region of interest of a patient;
a device for supporting improved respiration monitoring; and
a user interface configured to provide feedback on the quality of the respiration monitoring to the user based on the quality signal.

In yet further aspects of the present invention, there are provided corresponding methods, a computer program which comprises program code means for causing a computer to perform the steps of the methods disclosed herein when said computer program is carried out on a computer as well as a non-transitory computer-readable recording medium that stores therein a computer program product, which, when executed by a processor, causes the methods disclosed herein to be performed.

Preferred embodiments of the invention are defined in the dependent claims. It shall be understood that the claimed system, method and computer program have similar and/or identical preferred embodiments as the claimed device and as defined in the dependent claims and as disclosed herein.

The present invention is based on the idea to provide support for patients and clinical staff in respiration monitoring. Particularly, feedback on the quality of respiration monitoring shall be provided to allow for a better monitoring quality. In fact, it is an idea of the present invention to provide guidance for a patient (or other user) to come into an optimal position, orientation, posture and/or motion (stillness) such that respiration monitoring can be performed in an optimal manner.

To this end the patient is monitored by an imaging device such as a video camera, for example. The imaging device is particularly pointed at a region of interest of the patient, preferably a region which allows for motion due to respiration to be detected/measured. Recorded image frames are then provided to the device for supporting improved respiration monitoring. The input unit of the device is configured to obtain at least two image frames. The image frames need not be (directly) consecutive image frames, but may be image frames of (arbitrary) different points in time. The processing unit then analyzes said image frames. In particular, the processing unit is configured to estimate motion (of the patient depicted by the image frames), particularly in the region of interest, using said image frames. For example, the processing unit may be configured to detect motion in/for a plurality of pixels (voxels) and/or a group of pixels (voxels) of at least the region of interest for at least two image frames. Standard motion estimation algorithms may be applied to do so. The processing unit may further set a first (reference) direction and a second (reference) direction, which is substantially perpendicular to the first (reference) direction, (in the image plane of the set of images) and may project the motion detected onto the first direction and onto the second direction to estimate the amount of motion (of the patient) in the first direction and in the second direction. Alternatively, the detected motion may be filtered with respect to the first direction and the second direction. The amounts of motion in the first direction and/or second direction may be given by a motion signal (translation signal) corresponding to the first and/or second direction, respectively.

In the evaluation unit the amounts of motion (back and/or forth) in said two directions are compared with each other. In other words, both amounts are related to each other, wherein a relation (comparison) of said amounts comprises a difference of said amounts or a ratio of said amounts, for example. Based on the result of the comparison (relation) the evaluation unit determines a quality of respiration monitoring, particularly a value of a quality metric of the respiration monitoring. In other words, the processing unit is configured to convert the result of the comparison to a feedback metric. For example, the result of the comparison may be linked to a predetermined quality (metric value), as stored in a look-up table, for example. Based on the quality (particularly the quality metric value) the output unit provides a quality signal indicating the quality (metric value) of the respiration monitoring.

A user interface may use the quality signal to provide feedback on the quality of the respiration monitoring. The feedback may preferably be given in (near) real-time, i.e. during the respiration monitoring. The user interface may comprise a graphical user interface, such as a display or a touch screen, which may show the feedback as a (target) value and/or range. For example, the feedback may be provided in percent, wherein 100 percent indicates the highest possible quality metric value. In other words, the feedback may be provided in the form of a quality index. Similarly, the user interface may comprise a visual indicator in the form of one or more light sources, wherein said light sources may emit light in a predetermined color and/or in a predetermined (time) pattern based on the quality signal. In other word, the feedback may have the form of another non-numerical indication, such as a "traffic light", a (indicator in a) bulls-eye or audio cues. However, the user interface is not limited to these examples.

The feedback may be based on the patient's position, orientation, posture and/or motion (with respect to the imaging device). Information on the patient's position, orientation, posture and/or motion may be derived from the set of images provided by the imaging device itself or from other information sources.

By providing feedback to the patient during respiration monitoring the patient is provided with information on the quality of the monitoring and possibly advice to improve said monitoring directly. This is due to the fact that for contactless respiration monitoring measurement accuracy particularly improves when the patient remains static. Furthermore, by providing feedback the patient is also given something to focus on, which helps prevent distraction from remaining static (i.e. distraction which might cause unwanted motion, i.e. motion disturbing the respiration monitoring).

It shall be noted that the imaging device of the system may or may not be the same device used for contactless respiration (rate) monitoring. The imaging device of the system according to the present invention is primarily used to capture motion of the patient, particularly motion associated with respiration (inhaling and/or exhaling) of the patient. However, this does not necessarily imply that the images captured by said device are further used to gain information on the respiration rate, breathing rate or other vital signs associated with respiration. In fact, the imaging device of the system may be used to improve respiration monitoring while (another) radar device or camera may be used to perform measurements associated with respiration.

It is to be understood that in the context of the present application as disclosed herein the term "patient" is not limited to users suffering from illness, but may generally be used for any user or subject.

In an embodiment of the device for supporting improved respiration monitoring, the processing unit is configured to estimate the amount of motion of the patient by setting a motion vector reference point in one of the image frames and by determining a motion vector on the basis of a displacement of said vector reference point in another image frame. In particular, the processing unit is configured to estimate the amount of motion of the patient in the first direction by setting a first motion vector reference point in one of the image frames and by determining a first motion vector based on a displacement (i.e. translation) of said first vector reference point in the first direction in another image frame. Similarly, the processing unit may estimate the amount of motion of the patient in the second direction by using setting a second motion vector reference point in one of the image frames and by determining a second motion vector on the basis of a displacement of said second vector reference point in the second direction in the other image frame. Preferably, the first motion vector reference point and the second motion vector reference point are the same. Furthermore, it is preferred that the images, in which the first and second motion reference points are set, are the same and that the images, which are used to determine the displacement of said points are the same. This way it is ensured, that the motion amounts in the two directions are detected at the same time and at the same location. The motion vectors may also be called translation vectors or displacement vectors. In general, the processing unit may be configured to estimate the amount of motion of the patient by determining a series of motion vectors on the basis of a series of displacements of a vector reference point in a series of image frames. This way, particularly back and forth motion typical for breathing can be detected/captured.

In general, any available algorithm may be used to determine the first and second motion vector in the image frames. Respiratory effort may be tracked by using movement detection from optical flow, for example. Other examples of such algorithms include ProCor, kernel tracking and face tracking. In fact, some algorithms do not (directly) determine the displacement of a reference vector point. However, such displacement may be determined with any of these methods by determining a difference of detected positions in two separate images, for example. In this application, however, these positions are regarded as motion vector reference points.

In another embodiment, the amount of motion is the absolute value of motion integrated over time. Accordingly, the amount of motion may be given by the modulus (or a series of moduli) of the motion vectors), said modulus (series of moduli) being integrated over time, particularly the time between a first and a last image frame considered (i.e. between image frames to which the motion vector corresponds). More precisely, the amount of motion (or changes of motion) in the first (second) direction may be given by the modulus of the first (second) motion vector, the modulus being integrated over the time between the image frames to which the first (second) motion vector corresponds.

However, the amount of motion may be given by the absolute value of motion, particularly the modulus of the motion vector, only (i.e. without any integration over time).

In general, the integration over time may be omitted for the assessment of respiration measurement quality. A respiration signal is usually (a proxy of) position rather than speed. In other words, a measurement signal related to respiration monitoring is usually given in units of position. However, for camera-based measurements, speed (or a displacement of a motion vector reference point between frames) is generally easier to accurately measure than absolute position. Hence, for a camera-based respiration measurement, there is usually an integration step to go from speed to position, wherein the (position) signal may in turn be used for respiratory peak detection and/or rate computation. Hence, by including an integration step the quality of the final (position) signal as determined may represent the real quality in a more reliable way.

In another embodiment, the evaluation unit is configured to calculate the ratio of the amount of motion in the first direction and the amount of motion in the second direction and to determine the quality of the respiration monitoring based on said ratio. For example, the evaluation unit may be configured to calculate the ratio of a time integral of the absolute value of motion in the first direction and a time integral of the absolute value of motion in the second direction. More specifically, the evaluation unit may calculate the ratio of the time integral of the modulus of a motion vector in the first direction and the time integral of the modulus of a motion vector in the second direction. In other words, the evaluation unit may calculate the ratio of the time integral of the modulus of the translation signal corresponding to the first direction and the time integral of the modulus of the translation signal corresponding to the second direction.

If the value of said ratio is above or the same as a threshold value (the value being 1, for example), a respiration signal being based on the amount of motion in the first direction or, more generally, a respiration signal linked to the first direction (particularly to movements of the patient in the first direction) may be regarded as reliable. Said threshold value may depend on the type of respiration monitoring chosen and thus may be set in accordance with the type of respiration monitoring.

In general, a directional amplitude or any other indicator of the amount of motion of a patient (for example an indicator generated in intermediate steps in the determination of a motion vector) may be regarded as motion vector. Therefore, motion of the patient may also be represented by a series of directional amplitudes. Accordingly, the evaluation unit may be configured to compare the amount of motion in the first direction with the amount of motion in the second direction by determining a ratio of the directional amplitude in the first direction and the directional amplitude in the second direction, particularly by using the ratio of the moduli of said amplitudes, the moduli being integrated over time (similar to the motion vectors).

Motion vectors / directional amplitudes are not limited to images of a 2D camera but can also be applied to (determined from) image frames captured by 3D and/or thermal cameras, or radio frequency sensors.

In an embodiment of the device the first direction is substantially parallel to the direction of height of the patient and the second direction is substantially parallel to the direction of width of the patient (or vice versa). In particular, the processing unit may be configured to detect the direction of height and the direction of width of the patient (from the image frames, for example) and to compare said directions with the first direction and the second direction, respectively. Accordingly, the processing unit may be configured to detect a divergence of the first direction and the height direction and the second direction and the width direction, respectively. Furthermore, the processing unit may be configured to determine how the first and/or second direction need to be changed to match with the height and/or width direction, respectively, and to change/adapt said direction(s) accordingly.

In case the first direction is (substantially) the same as the height direction and the second direction is (substantially) the same as the height direction, it is expected that respiratory movements (particularly movements of the chest and belly) are (substantially) limited to the second direction. Therefore, if the evaluation unit determines (upon comparison) that the amount of motion in the second direction is much higher than the amount of motion in the second direction the quality of the respiration monitoring may be determined to be high. In fact, depending to the comparison of both motion amounts the processing unit may determine a quality index, wherein the quality index is higher, the higher the amount of motion in the second direction is with respect to the first direction.

In yet another embodiment, input unit, the processing unit, the evaluation unit and the output unit are configured to work in real-time. Accordingly, obtaining the image frames and providing the quality signal is quasi-instantaneous. In other words, the device is configured to perform real-time processing. In case the device is part of a system comprising a user interface this allows for real-time feedback about the respiration monitoring quality to the user.

In a further embodiment, the processing unit is configured to generate a respiration signal from the set of image frames, wherein the processing unit is configured to filter the respiration signal. The respiration signal may indicate a number of breaths of the patient over a predetermined time. In fact, the respiration signal may be a signal indicating the amount of motion in the first and/or second direction (and accordingly indicating a number of breaths in a predetermined time). Accordingly, the respiration signal may also be called motion signal and the motion signal may be subject to filtering, particularly frequency filtering.

In particular, the processing unit may be configured to estimate the number of breaths of the patient in a time span between two of the set of image frames by using the estimated amount of motion of the patient in the first direction and/or second direction (in said time span), for example by using a motion vector series. However, the respiration signal may be determined otherwise from the set of image frames. The respiration signal may then further be filtered. For example, the processing unit may be configured to filter the respiration signal with respect to a predetermined frequency band, i.e. to filter out (and/or cut off) predetermined frequencies. To this end a bandpass filter allowing frequencies in the expected vital sign range, i.e. 5 to 60 breaths per minute (for adults), may be used. Accordingly, (the signal corresponding to) the amount of motion in the first and/or second direction may be filtered.

Therefore, to determine the quality of the respiration monitoring more accurately, the evaluation unit may be configured to compare the filtered amount of motion in the first direction with the (filtered) amount of motion in the second direction and to determine a quality of respiration monitoring based on said comparison.

In another embodiment the evaluation unit is configured to compare the amount of motion in the first direction with a first threshold and/or to compare the amount of motion in the second direction with a second threshold, wherein the evaluation unit is configured to determine a gain factor based on any of said comparisons, respectively. In particular, the evaluation unit may be configured to compare the amount of motion in the first direction with the first threshold value und to determine a first gain factor based in this comparison. Similarly, the evaluation unit may be configured to compare the amount of motion in the second direction with the second threshold value und to determine a second gain factor based in this comparison. In particular, it may be determined if and to what extent the amounts of motion comply with said threshold values, respectively. A corresponding gain factor may be determined then. For example, if the amount of motion in the first direction complies with the first threshold value, particularly is below the first threshold value, the gain factor may be set to a high value (low value). On the other hand, if the amount of motion in the first direction does not comply with the first threshold value, particularly is the same or above the first threshold value, the gain factor may be set to a low value (high value). In general, the gain factor may be any numerical value, preferably normalized to the interval [0, 1].

In particular, the evaluation unit may be configured to determine the quality of the respiration monitoring based on any of said gain factor(s). In fact, as described above, the gain factor represents a measure of for the amount of motion. In general, it can be expected that the higher the amount of motion in the direction the chest of the patient moves during respiration is (as detected), the better is the quality of the respiration monitoring. However, a very large motion may not stem from respiratory movement but may be caused by other motions of the patient. Accordingly, a very large amount of motion is likely to prevent accurate measurement. In other words, a very large amount of motion as detected/measured is an indicator for a low quality of the respiration monitoring (regardless of the comparison of the amounts of motion). Therefore, the application of the gain factor to the quality determined (and hence to the quality signal and the feedback provided by the system) is advantageous.

In an embodiment of the system for supporting improved respiration monitoring, the imaging device comprises any of a camera, particularly a video camera; a radar sensor, particularly a Doppler radar sensor; an ultrasonic imaging device; a thermal camera, particularly a microbolometer camera; a laser Doppler imaging device; a speckle vibrometry unit; and a depth camera, particularly a time-of-flight camera.

For example, if a patient's face is visible, thermal infra-red cameras may detect temperature differences and changes in front of the nose/mouth region. Depth cameras allow obtaining 3D images. Hence, time-of-flight (ToF) cameras allow for depth sensing. However, depth reconstruction may also be achieved by using multiple (video) cameras (stereo principle). Irrespective of the kind of imaging device mentioned, respiration measurement using any of these devices benefits from limited body motion and hence the support given by the device for supporting improved respiration monitoring.

In another embodiment of the system, the feedback provided by the user interface further comprises advice for enhancing the quality of the respiration monitoring, the advice relating to the patient's position, orientation, posture and/or motion. The advice may comprise a signal to the patient to stand still or to move to a location where he/she is better visible for the imaging device of the system. In particular, the advice may comprise an instruction for the patient to change his/her position, orientation and/or posture such that the first direction is substantially parallel to the direction of height of the patient and the second direction is substantially parallel to the direction of width of the patient (or vice versa). Accordingly, the device may comprise an instruction for the patient to turn by 90° or to turn around, to stand upright, to lay down or the like.

To provide said feedback the device for supporting improved respiration monitoring, particularly the processing unit of said device may be configured to detect the direction of height and the direction of width of the patient and to compare said directions with the first direction and the second direction respectively. Accordingly, the processing unit may be configured to detect a divergence of the first direction and the height direction and the second direction and the width direction, respectively. Furthermore, the processing unit may be configured to determine how the first and/or second direction need to be changed to match with the height and/or width direction, respectively, and to adapt said direction(s) accordingly. Similarly, the processing unit may be configured to determine how the height and/or width direction of the patient would need to be changed to match with first and/or second direction, respectively. Based on that, the processing unit may be configured to provide a signal comprising an instruction for the patient how to change his/her position, orientation, posture and/or motion such that the first direction is the same as the height direction and/or the second direction is the same as the width direction. Based on said signal the user interface of the system may provide the above-mentioned advice.

In yet another embodiment of the system, the user interface is configured to provide the feedback in real-time, and/or the user interface is configured provide the feedback as visual, audio and/or tactile feedback.

Since the device for supporting improved respiration monitoring may be configured to work in real-time, the quality signal may be provided in real-time. Accordingly, the user interface of the system is capable to provide feedback on the quality of respiration monitoring in real-time. Hence, health professionals and patients may get immediate feedback on the quality of an ongoing respiration monitoring and may intervene immediately to improve said monitoring. This allows clinical diagnoses to be made faster, more reliably and more precisely.

The feedback of the system may be provided in different ways and thus may be adapted to the patient's health status and monitoring method chosen. For example, if the patient is blind or if clinical staff may not be able to supervise a monitor because of other tasks to be done, the user interface may comprise a speaker to provide audio feedback on the quality of the respiration monitoring. Similarly, the user interface may comprise a screen indicating an optimal monitoring position and an actual position of the patient. Accordingly, the patient can see whether his/her position is optimal or not and how to move to come into the optimal position for respiration monitoring. As another example, the patient may stand on a platform, capable of vibrating, during respiration monitoring, wherein the platform vibrates when the patient's position, orientation, posture and/or motion does not allow for high quality monitoring.

Preferably, the feedback is provided in a continuous manner. However, the feedback may also be provided in (time) intervals, particularly in regular (time) intervals.

### BRIEF DESCRIPTION OF THE DRAWINGS

These and other aspects of the invention will be apparent from and elucidated with reference to the embodiment(s) described hereinafter. In the following drawings:
Fig. 1 shows a schematic diagram of an embodiment of a device according to the present invention,
Fig. 2 shows a patient 50 during respiration and orthogonal reference directions according to the present invention,
Fig. 3 shows a flow chart of a first embodiment of a method according to the present invention,
Fig. 4 shows a flow chart of an embodiment of a step of the first embodiment of the method according to the present invention,
Fig. 5 shows a first set of diagrams of translation signals indicating an amount of motion in a horizontal direction and in a vertical direction, respectively, and a comparison thereof.
Fig. 6 shows a second set of diagrams of translation signals indicating an amount of motion in a horizontal direction and in a vertical direction, respectively, and a comparison thereof.
Fig. 7 shows a third set of diagrams of translation signals indicating an amount of motion in a horizontal direction and in a vertical direction, respectively, and a comparison thereof.
Fig. 8 shows a schematic diagram of an embodiment of a system according to the present invention,
Fig. 9 shows various diagrams illustrating feedback on the quality of the respiration monitoring as provided by the system according to the present invention, and
Fig. 10 shows diagrams illustrating advice for supporting the respiration monitoring as performed by the system 1 of the present invention.

### DETAILED DESCRIPTION OF THE INVENTION

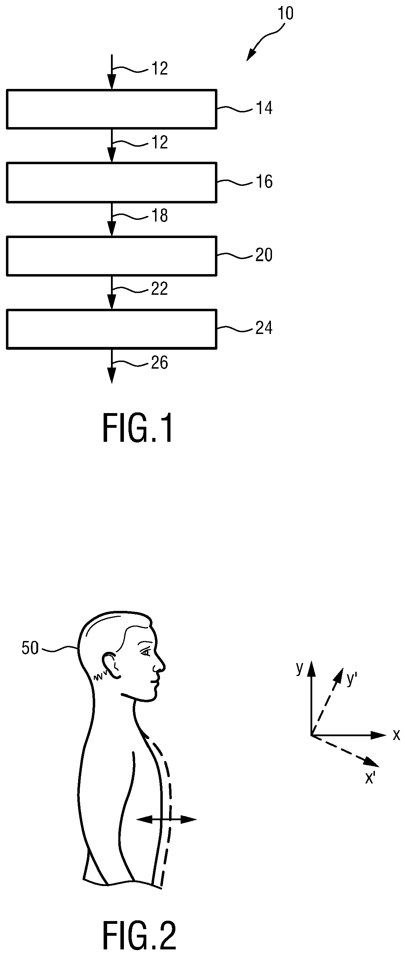

Fig. 1 shows a schematic diagram of an embodiment of a device 10 according to the present invention. The device 10 comprises an input unit an input unit 14, a processing unit 16, an evaluation unit 20 and an output unit 24. The device 10 may comprise circuitry, e.g. a processor, processing circuitry, a computer, dedicated hardware, etc. that carries out the functions of the device. In another embodiment, separate units or elements may be used that together represent the circuitry.

In this embodiment, the input unit 14 is configured to obtain a set of image frames 12, particularly a stream of images, of a region of interest of a patient 50. The set of image frames 12 may be obtained from an imaging device or from a storage, for example. For this purpose the input unit 14 may be directly or indirectly (e.g. via a network or bus) coupled or connected to the imaging device or the storage. The input unit 14 may thus e.g. be a (wired or wireless) communication interface or data interface, such as a Bluetooth interface, Wi-Fi interface, LAN interface, HDMI interface, direct cable connection, or any other suitable interface allowing data transfer to the device 10. The set of image frames 12 obtained may particularly show the chest region of the patient 50. The set of image frames 12 may be provided to the processing unit 16.

In the processing unit 16 a part or all of the region of interest may be used to determine motion of the patient between at least two (not necessarily consecutive) image frames 12. In particular, the processing unit 16 may analyze the image frames 12 (particularly in the chest region) and may estimate an amount of motion of the patient's chest in two directions, the directions being substantially orthogonal to each other. In particular, the processing unit 16 estimates an amount of motion of the chest in a first direction and in a direction perpendicular to the first direction (second direction). The amount of motion may be provided by the distance the chest moves in the first direction and in the second direction, respectively. Motion data 18, comprising the estimation of the amount of motion in both directions (for example, a translation signal indicating motion in the first direction and a translation signal indicating motion in the second direction), is then provided to the evaluation unit 20, which compares the amount of motion in both directions with each other. In other words, the evaluation unit 20 compares the distance the chest hast moved in the first direction with a distance the chest has moved in the second direction. Based on said motion comparison the evaluation unit 20 determines a quality metric 22 of the patient's monitoring. Bothe the processing unit 16 and the evaluation unit 20 may be any kind of means configured to process the image frames 12, to estimate an amount of motion of the patient in a first direction and in a second direction by use of said frames and to determine a quality of the respiration monitoring by comparison of said amounts of motion. The processing unit 16 and the evaluation unit 20 may be implemented in software and/or hardware, e.g. as a programmed processor, computer, laptop, PC, workstation, etc.

Using the quality/quality metric 22 of the respiration monitoring determined by the evaluation unit 20 the output unit 24 provides a quality signal 26. The output unit 24 may generally be any interface that provides the generated signal, e.g. transmits it to another device or provides it for retrieval by another device, e.g. transmits it to another computer or transfers it directly to a user interface 40 for displaying it. It may thus generally be any (wired or wireless) communication or data interface.

Fig. 2 shows a patient 50 during respiration and orthogonal reference directions. The solid line shows the patient in a state in which he/she has exhaled. The dashed line indicates a state in which the patient has inhaled, i.e. in a state in which the lungs are expanded, the chest cavity is enlarged and thus the rib cage is outward. In fact, respiratory motion (due to inhalation/exhalation) is expected to mainly be observable as transversal motion when a patient is seated or standing. This is indicated by the double arrow in Fig. 2, which is parallel to the x-axis. On the other hand, there is no motion or at least not much motion in the y-direction expected when said patient inhales or exhales. Therefore, a metric for respiratory motion, i.e. motion of the patient's body (particularly chest and belly), may be the integral of the absolute measured vertical translation (or `travelled distance'), i.e. the translation in y-direction, in a certain moving window, divided by that in the horizontal direction (x-direction). A value of this ratio significantly above 1 would indicate that the vertical motion is stronger than the horizontal motion. In this case, the motion detected may not stem from respiration, but from other movements of the patient. In other words, respiration monitoring should not be trusted if said ratio is above 1. The validity of said ratio is strongest if the x-axis and the y-axis are chosen to be (substantially) parallel to the patient's direction of width and direction of height, wherein the patient's direction of width may be defined by a straight line between the patient's left and right shoulder tip, for example. Similarly, the patient's height direction may be defined by a straight line indicating head-to-toe distance.

In general, another set of (preferably orthogonal) directions may be chosen, for example as directions not being parallel and perpendicular to the height of the patient, such as directions x' and y' as shown in Fig. 2.

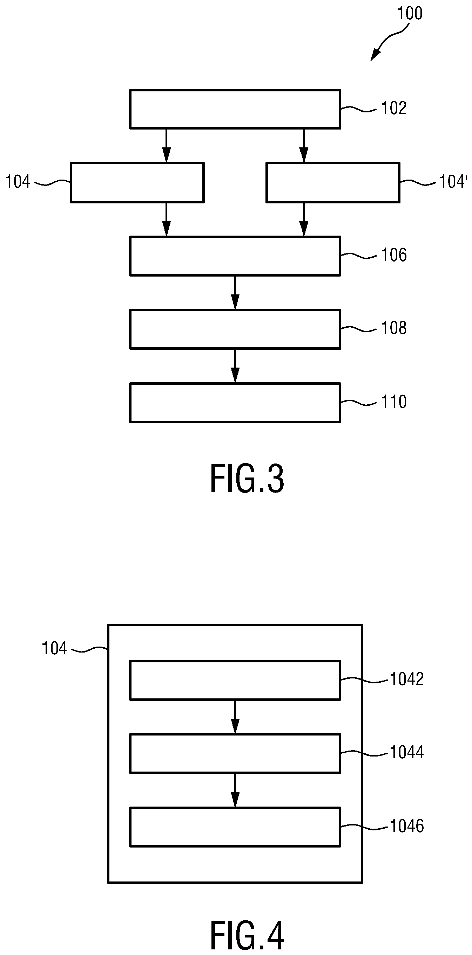

Fig. 3 shows a flow chart of a first embodiment of a method 100 according to the present invention. The steps of the method may be carried out by the device 10, wherein the main steps of the method are carried out by the processing unit 16 and the evaluation unit 20. The method may e.g. be implemented as computer program running on a computer or processor.

In a first step 102 of the method 100 a set of image frames 12 of a region of interest of the patient are obtained (either received or retrieved). The image frames 12 may be obtained directly from a camera or from a storage, such as a buffer or memory or image repository, e.g. a patient record stored in a hospital's document management system, e.g. via a wired or wireless network.

In second steps 104 and 104' the image frames are analyzed with respect to motion of the patient in the region of interest in two different directions. In particular, in step 104 there is estimated an amount of motion of the patient in a first direction and step 104' there is estimated an amount of motion of the patient in a second direction, wherein the second direction is substantially perpendicular to the first direction. For this purpose, generally any available algorithm may be used. Examples of motion estimation algorithms comprise ProCor, optical flow, kernel tracking and face tracking. Using these algorithms motion vectors in the first and second direction, respectively, may be determined. In this embodiment of the method 100 steps 104 and 104' are performed in parallel. However, said steps may likewise be performed in consecutive order with step 104 being followed by step 104' or vice versa.

Once the amount of motion in the first and in the second direction, respectively, is estimated, both amounts are compared with each other in step 106. In step 108 a quality metric of respiration monitoring is determined based on the comparison in step 106. In step 110 a quality signal is generated based on the quality metric and provided for further processing.

Fig. 4 shows a flow chart of an embodiment of step 104 of the first embodiment of the method 100. In this embodiment step 104 is divided into three sub-steps 1042, 1044 and 1046. As explained above, in step 104 there is estimated an amount of motion of the patient in the first (reference) direction. In particular, in a first sub-step 1042 there is set a motion vector reference point in one of the image frames. A motion vector is determined based on a displacement/translation of said vector reference point in another (consecutive) image frame. In sub-step 1044 the absolute value of the motion vector is determined. In other words, the modulus of the motion vector is determined. Subsequently, the modulus of the motion vector is integrated over time.

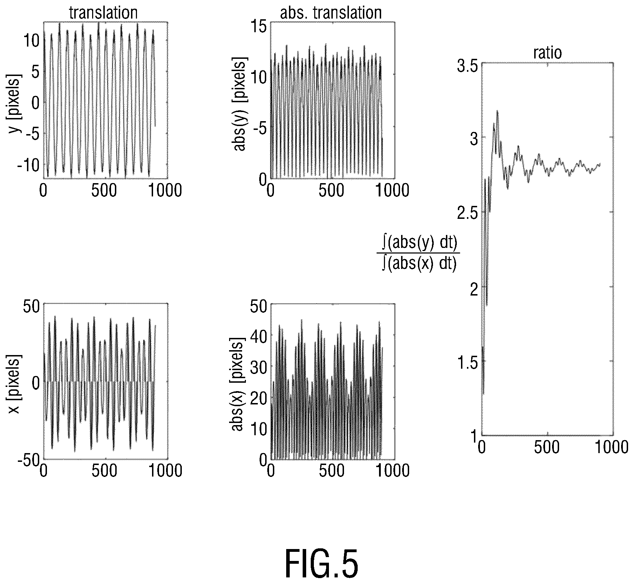

Figs. 5, 6 and 7 each show sets of diagrams of translation signals indicating an amount of motion in a horizontal direction and in a vertical direction, respectively, and a comparison thereof. In particular, the figures show an amount of motion of a part of a patient, the part being represented by a motion vector reference point, for example. The signals shown indicating the amounts of motion in the different directions are derived from a time series of horizontal and vertical motion vectors (i.e. from a set comprising a plurality of image frames). In the upper left diagram (of each of said figures) the horizontal motion of the (part of the) patient is represented by a horizontal translation signal. In the upper middle diagram the absolute value of the horizontal translation signal is shown. The lower left and lower middle diagram show the vertical translation signal and the absolute values thereof, respectively. As can be seen from the diagrams in Fig. 5, particularly those in the middle, the amplitude of the vertical translation signal is significantly higher than the amplitude of the horizontal translation signal. This implies that the motions of the patient as detected rather go in a vertical direction than in a horizontal direction. In the given case, the quality metric for determining the quality of the patient's respiration monitoring is given by the ratio of the time integral over the absolute translation in the vertical direction and the time integral over the absolute translation in the horizontal direction. This ratio, i.e. the quality metric, is shown in the diagram on the right. As can be seen from this diagram, the value of the quality metric is significantly above 1. In this case, it can be assumed that it is likely that respiration can be retrieved from the vertical signal (in a reliable manner).

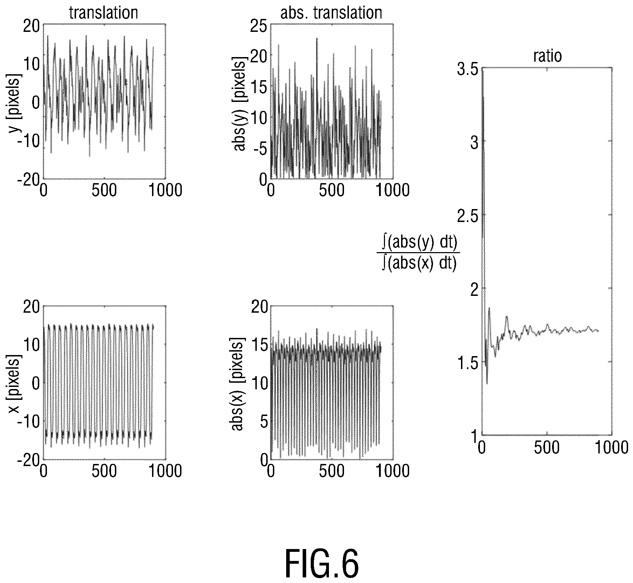

Fig. 6 shows in the upper left diagram the horizontal motion of a (part of the) patient, i.e. a (horizontal) translation signal. In the upper middle diagram the absolute value of the horizontal translation signal is shown. The lower left and lower middle diagram show the vertical translation signal and the absolute values thereof, respectively. As can be seen, the horizontal translation signal is very different from the vertical translation. However, the amplitudes of said signal are similar. On the right, a diagram showing the ratio of the time integral over the absolute translation in the vertical direction and the time integral over the absolute translation in the horizontal direction, said ratio representing the quality (metric) for respiration monitoring is shown. The metric is shown to be above a value of 1. Hence, there is a reasonable likelihood that a reliable determination of features regarding respiration can be retrieved from the vertical signal.

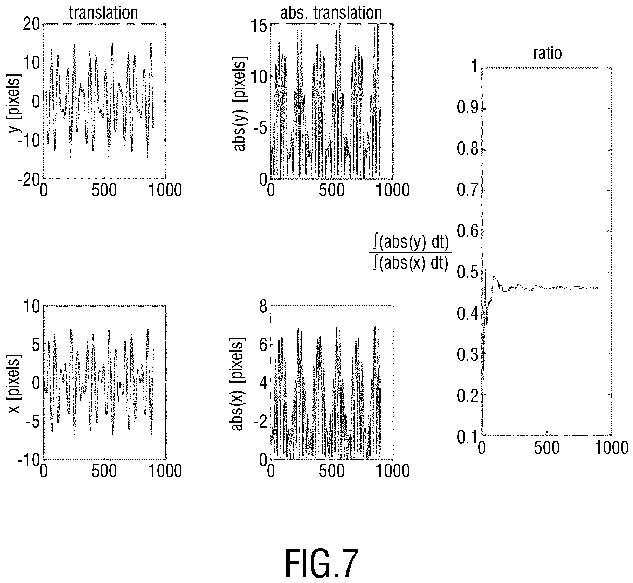

Fig. 7 shows in the upper left diagram the horizontal motion of a (part of the) patient, i.e. a (horizontal) translation signal. In the upper middle diagram the absolute value of the horizontal translation signal is shown. The lower left and lower middle diagram show the vertical translation signal and the absolute values thereof, respectively. According to the measurement signals shown in the diagrams the (absolute) horizontal translation signal dominates the (absolute) vertical translation signal. The quality metric, given by the ratio of the time integral over the absolute translation in the vertical direction and the time integral over the absolute translation in the horizontal direction, is below 1. Therefore, it is unlikely that respiration monitoring can be retrieved from the vertical translation signal in a reliable manner.

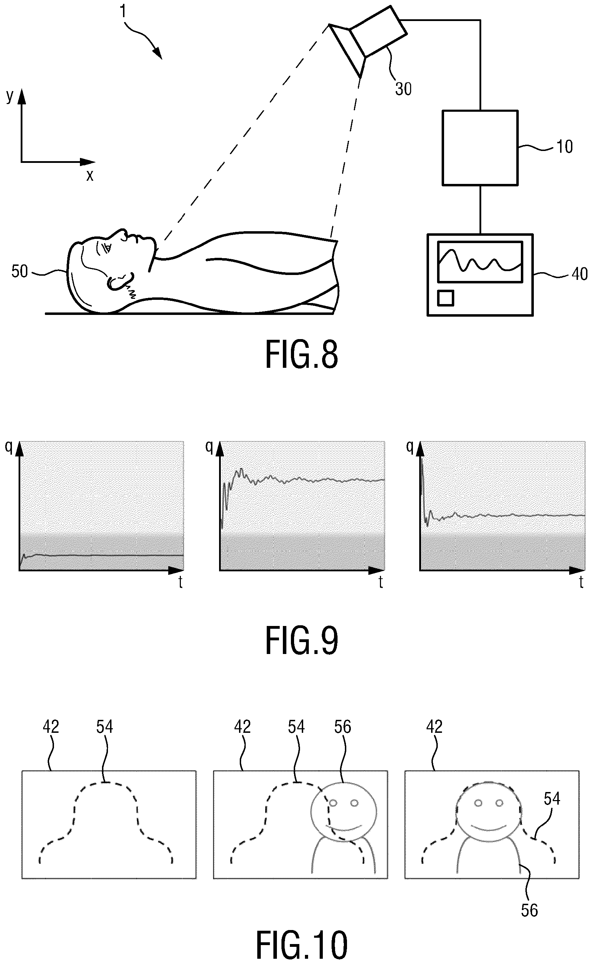

Fig. 8 shows a schematic diagram of a first embodiment of a system 1 according to the present invention. In this embodiment the system 1 comprises a device 10 for supporting improved respiration monitoring, in an imaging device 30, and a user interface 40.

The imaging device 30 is configured to perform respiration monitoring of the patient and comprises a video camera in this embodiment. However, in general, the imaging device 30 may be a radar sensor, an ultrasonic imaging device, a thermal camera, a laser Doppler imaging device, a speckle vibrometry unit, or a time-of-flight camera as conventionally used for acquiring image data of a subject, e.g. of a certain body region of a patient. The imaging device 30 may e.g. be implemented as or in a processor or computer, in software and/or hardware. For instance, a programmed processor may be included in the imaging device 30, which may execute a computer program that may be stored in a memory that is accessed by the processor.

In this embodiment, the video camera is directed to the patient's chest to capture an image stream comprising a set of image frames 12 of the patient for a predetermined period of time. The imaging device 30 provides the image frames 12 to the device 10, which analyzes the image frames 12 and provides a quality signal 26 indicating the quality of the respiration monitoring of the imaging device 30.

The user interface 40, a touch-screen in this embodiment, then provides feedback on the quality of the respiration monitoring based the quality signal. In particular, the touch-screen is configured to display the quality in terms of a quality index, for example. Alternatively or additionally, the feedback on the quality of the respiration signal may be provided by the user interface 40 in a color-coded manner.

Fig. 9 shows various diagrams illustrating feedback on the quality of the respiration monitoring as provided by the system 1 according to the present invention. In particular, the diagrams show a quality index q over respiration monitoring time t. The quality index q indicates the quality of respiration monitoring by the imaging device 30 of the system 1. In the left diagram the quality index is quite low and is only located in region which is shaded in dark gray. This implies, that the quality of the respiration monitoring is low and therefore that respiration is monitored in a non-reliable manner. In an embodiment, the dark-shaded area may be shaded in red and the light shaded area may be shaded in green to better visualize the implication of the quality index shown.

In the diagram in the middle and on the right the quality index q can be regarded to represent a reliable respiration monitoring, since the index is clearly in the light shaded area, i.e. the quality index is above a predetermined threshold (indicated by the transition from the dark-shaded area to the light-shaded area). However, as the values of q are generally higher in the diagram in the middle than in the diagram on the right, the quality of the respiration monitoring corresponding to the diagram in the middle can be regarded to be higher than the quality of the respiration monitoring corresponding to the diagram on the right.

Fig. 10 shows diagrams illustrating advice for supporting the respiration monitoring as performed by the system 1 according to the present invention. In particular, Fig. 10 shows a screen of a user interface 40 of the system in three different situations. The screen depicted on the left shows a position template (dashed line) corresponding to an optimal position (and orientation) of the patient's head and shoulder, the indicated position (and orientation) allowing for optimal respiration monitoring. Accordingly, said screen of the user interface may be regarded as a guide or advisory means for the patient where to position himself/herself. Further, as depicted in the screen in the middle, the user interface may further provide feedback on the actual position of the patient (solid line). In other words, the feedback screen may show the camera view of the patient (abstracted by the solid line) overlaid with an outline of the optimal patient position. As can be seen from the screen in the middle, the actual position of the patient does not correspond to the optimal position indicated by the template. However, from the display of both positions the patient is advised how to move to take up the optimal position. The screen on the right shows the position of the patient and the position template in congruence. Accordingly, the right screen displays a position in which the patient has the optimal position for respiration monitoring.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Device (10) for supporting improved respiration monitoring, the device (10) comprising:
an input unit (14) configured to obtain a set of image frames (12) of a region of interest of a patient (50);
a processing unit (16) configured to analyze the set of image frames (12) to estimate an amount of motion of the patient (50) in a first direction and in a second direction, wherein the second direction substantially perpendicular to the first direction,
an evaluation unit (20) configured to compare the amount of motion in the first direction with the amount of motion in the second direction and to determine a quality of respiration monitoring based on said comparison; and
an output unit (24) configured to provide a quality signal (26) indicating the quality of the respiration monitoring.

2. Device (10) as claimed in claim 1,
wherein the processing unit (16) is configured to estimate the amount of motion of the patient by setting a motion vector reference point in one of the image frames and by determining a motion vector on the basis of a displacement of said vector reference point in another image frame.

3. Device (10) as claimed in claim 1 or 2,
wherein the amount of motion is the absolute value of motion integrated over time.

4. Device (10) as claimed in any of the preceding claims,
wherein the evaluation unit (20) is configured to calculate the ratio of the amount of motion in the first direction and the amount of motion in the second direction and to determine the quality of the respiration monitoring based on said ratio.

5. Device (10) as claimed in any of the preceding claims,
wherein the first direction is substantially parallel to the direction of height of the patient (50) and wherein the second direction is substantially parallel to the direction of width of the patient (50).

6. Device (10) as claimed in any of the preceding claims,
wherein the input unit (14), the processing unit (16), the evaluation unit (20) and the output unit (24) are configured to work in real-time.

7. Device (10) as claimed in any of the preceding claims, wherein the processing unit (16) is configured to generate a respiration signal from the set of image frames, wherein the processing unit (16) is configured to filter the respiration signal.

8. Device (10) as claimed in any of the preceding claims,
wherein the evaluation unit (20) is further configured to compare the amount of motion in the first direction with a first threshold and/or to compare the amount of motion in the second direction with a second threshold,
wherein the evaluation unit (20) is configured to determine a gain factor based on any of said comparisons.

9. Device (10) as claimed in claim 8,
wherein the evaluation unit (20) is configured to determine the quality of the respiration monitoring based on the gain factor.

10. System (1) for supporting improved respiration monitoring, the system comprising:
an imaging device (30) configured to capture a set of image frames of a region of interest of a patient;
a device (10) as claimed in any of claims 1 to 9; and
a user interface (40) configured to provide feedback on the quality of the respiration monitoring to the user based on the quality signal.

11. System (1) as claimed in claim 10,
wherein the imaging device (30) comprises any of a camera, particularly a video camera; a radar sensor, particularly a Doppler radar sensor; an ultrasonic imaging device; a thermal camera, particularly a microbolometer camera; a laser Doppler imaging device; a speckle vibrometry unit; and a depth camera, particularly a time-of-flight camera.

12. System (1) as claimed in claim 10 or 11,
wherein the feedback further comprises advice for enhancing the quality of the respiration monitoring, the advice relating to the patient's position, orientation, posture and/or motion.

13. System (1) as claimed in any of claims 10 to 12,
wherein the user interface (40) is configured to provide the feedback in real-time, and/or
wherein the user interface (40) is configured provide the feedback as visual, audio and/or tactile feedback.

14. Method for supporting improved respiration monitoring, the method comprising:
obtaining a set of image frames of a region of interest of a patient;
analyzing the set of image frames to estimate an amount of motion of the patient in a first direction and in a second direction, wherein the second direction is substantially perpendicular to the first direction,
comparing the amount of motion in the first direction with the amount of motion in the second direction and determining a quality of respiration monitoring based on said comparison; and
providing a quality signal indicating the quality of the respiration monitoring.

15. Computer program comprising program code means for causing a computer to carry out the steps of the method as claimed in claim 14 when said computer program is carried out on the computer.
